# EUROPEAN PATENT APPLICATION

(11) **EP 1 421 955 A1**
(43) Date of publication of application: **26.05.2004**
(21) Application number: 02762767.8
(22) Date of filing: 09.08.2002
(51) Int. Cl.: A61K 47/32, A61K 31/48, A61K 31/4985, A61P 25/16

(54) **PERCUTANEOUS ABSORPTION PREPARATIONS**

(30) Priority: 10.08.2001 JP 2001243787
(71) Applicant: HISAMITSU PHARMACEUTICAL CO., INC., Tosu-shi Saga 841-0017 (JP)
(72) Inventor: TERAHARA, Takaaki, Hisamitsu Pharmaceutical Co.Inc, Tsukuba-shi, Ibaraki 305-0856 (JP); AIDA, Kazunosuke, Hisamitsu Pharmaceutical Co.Inc, Tsukuba-shi, Ibaraki 305-0856 (JP); HIGO, Naruhito, Hisamitsu Pharmaceutical Co., Inc., Tsukuba-shi, Ibaraki 305-0856 (JP); SATO, Shuji, Hisamitsu Pharmaceutical Co., Inc., Tsukuba-shi, Ibaraki 305-0856 (JP)
(74) Representative: Ede, Eric
(86) International application number: PCT/JP2002/008165
(87) International publication number: WO 2003/013611

(57) **Abstract**

It is intended to provide external preparations for percutaneous application and compositions therefor whereby a hardly soluble drug such as a drug having an ergoline skeleton can be effectively absorbed via the skin into the blood under circulation while avoiding side effects on the digestive system observed in case of oral administration or side effects on the central system caused by a rapid increase in the concentration in the blood and a high adhesiveness to the skin can be established. This object can be established by composition characterized by containing a copolymer having N-vinyl-2-pyrrolidone as one of its constituents and percutaneous absorption preparations characterized by having a drug-containing adhesive layer containing such a composition and a support layer.

## Description

### [Detailed Description of the Invention]

### [Technical Field]

The invention relates to a composition containing a copolymer as a base wherein it has N-vinyl-2-pyrrolidone as one of its constituents as a device for a percutaneous absorption of a hardly soluble drug such as a drug having an ergoline skeleton represented by pergolide, bromocriptine, lisuride and the like, and a percutaneous absorption preparation which improves a skin permeability of said drug using said composition in an adhesive layer.

### [Background Art]

Adrug having an ergoline skeleton represented by pergolide, bromocriptine, lisuride and the like is dopaminergic, and is used for a parkinsonism patient in a single treatment or a combination treatment with levodopa. These preparations used in medical care are oral preparations and have the following problems from the characteristics that the drugs andparkinsonism patients have.
a) They have a lot of side effects toward the digestive tract due to a dopamine-like action to the digestive tract.
b) They are low in bioavailability due to an easy vulnerability to the first pass effect in the liver.
c) Adigestive function of parkinsonism patients is reduced in many cases, and there are cases in which a drug absorption in an oral administration is reduced.

As a means to solve these problems, several percutaneous absorption preparations have been proposed. For example, as to pergolide, a percutaneous patch dosage form titratable for administration of a therapeutic substance is proposed in JP A 6-9379 wherein pergolide is shown as a drug of its possible application. In JP 11-507361 A, a percutaneous absorption preparation of pergolide is proposed, and the skin permeation velocity attained thereby and a permeation promoter are described. In JP 2000-514053 A, a percutaneous absorption preparation is proposed in which pergolide or another drug is blended. In WO 99/59558, a reservoir-type percutaneous absorption preparation containing pergolide is proposed. Further as to lisuride, a percutaneous absorption preparation of lisuride is proposed in JP 4-506958 A, and an absorption promoter suitable therefor is described.

However, although the problems of the oral preparation above described are solved in these percutaneous absorption preparations, there is no specific description on skin irritating properties, stability of the drugs and the preparations, and physical properties of the preparations, and a percutaneous absorption preparation of pergolide or lisuride actually used in a clinical field has not yet been developed. As one of its reasons, it is cited that the solubility of a drug having an ergoline skeleton toward a base used in the percutaneous absorption is very low.

Therefore, the development of a percutaneous absorption preparation for administration of a poorly-soluble drug toward a base like a drug having the ergoline skeleton is desired.

Consequently, the problem of the invention is to provide external preparations for percutaneous application and compositions therefor whereby a hardly soluble drug such as a drug having an ergoline skeleton can be efficiently absorbed via the skin into the blood under circulation while avoiding side effects on the digestive system observed in case of oral administration or side effects on the central system caused by a rapid increase in the concentration in the blood and a high adhesiveness to the skin can be established.

### [Disclosure of the Invention]

The inventors of the invention made extensive researches to solve the above problems, focused on the fact that by using a base in which a hardly soluble drug such as a drug having an ergoline skeleton has been dissolved in a solvent which provides extremely high solubility with said drug, the skin permeability of said drug is remarkably improved, and accomplished the invention by finding a composition containing as a solvent a copolymer having particular constituents.

Namely, the invention relates to a composition accelerating percutaneous absorption of a drug, characterized in that said composition comprises a copolymer having N-vinyl-2-pyrrolidone as one of its constituents.

Further, the invention relates to the above composition, characterized in that said composition comprises a copolymer having N-vinyl-2-pyrrolidone as one of its constituents and that the drug is a drug having an ergoline skeleton.

Also, the invention relates to the above composition, characterized in that the copolymer is a copolymer of a (meth)acrylic acid derivative and N-vinyl-2-pyrrolidone.

Further, the invention relates to the above composition, characterized in that the (meth)acrylic acid derivative is 2-ethylhexyl acrylate.

Also, the invention relates to the above composition, characterized in that the (meth)acrylic acid derivative is 2-ethylhexyl acrylate and 1,6-hexaneglycol dimethacrylate.

Further, the invention relates to the above composition, characterized in that the drug having an ergoline skeleton is pergolide and/or drugly acceptable acid-addition salts thereof.

Also, the invention relates to the above composition, characterized in that the acid-addition salt of pergolide is pergolide mesylate.

Further, the invention relates to the above composition, characterized in that the drug having an ergoline skeleton is bromocriptine and/or drugly acceptable acid-addition salts thereof.

Also, the invention relates to the above composition, characterized in that the acid-addition salt of bromocriptine is bromocriptine mesylate.

Further, the invention relates to the above composition, characterized in that it contains an organic acid and/or drugly acceptable acid-addition salts thereof.

Also, the invention relates to the above composition, characterized in that the organic acid is acetic acid, propionic acid, lactic acid or salicylic acid.

Further, the invention relates to the above composition, characterized in that the organic acid is acetic acid or lactic acid.

Also, the invention relates to a percutaneous absorption preparation, characterized in that the preparation comprises a drug-containing adhesive layer comprising any one of the above compositions and a support layer.

By making the composition of the invention contain at least one component from acetic acid, propionic acid, lactic acid and salicylic acid and/or drugly acceptable salts of these acidic substances, a skin permeation effect of the drug is further improved and preferably a higher percutaneous absorption of the drug is attained.

The composition of the invention has no skin irritating property and can dissolve even a hardly soluble drug. Therefore, by use of said composition, a preparation containing the hardly soluble drug can be prepared without skin irritating properties and without problems of the stability of a drug and a preparation, and of physical properties of the preparation.

### [Brief Description of Drawings]

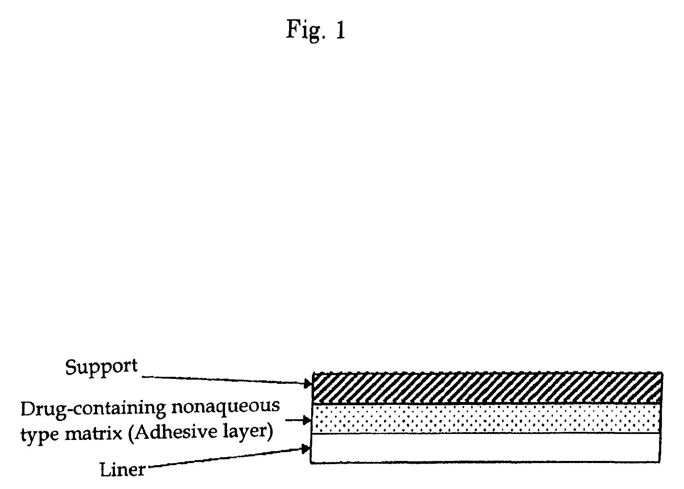

Fig. 1 is an example of the constitution of percutaneous absorption preparations of the invention.

### [Mode for carrying out the Invention]

Percutaneous absorption preparations can be prepared by applying a composition of the invention to an adhesive preparation and a liniment.

In the following, the composition and form in an adhesive layer of an adhesive preparation of the invention are explained. The percutaneous absorption preparation used in the invention is preferably in the form consisting of an adhesive layer containing a drug and a backing support therefor as shown in Fig. 1. The adhesive layer has an adhesive strength to maintain a therapeutically effective area on the skin surface for at least not less than 12 hours.

As a drug used in the adhesive layer of an adhesive preparation of the invention, its species is not particularly limited as long as it is hardly soluble, percutaneously absorbable and in addition, a drug having an ergoline skeleton; Illustrative are, for example, pergolide mesylate, bromocriptine mesylate and lisuride maleate.

Further, these drugs may be used alone or in a combination of two or more species, and any form of drug such as those in a molecular form, an inorganic salt or an organic salt is reasonably included. Also, considering a sufficient permeable amount as the adhesive preparations and irritating properties to the skin such as rubor, drugs can be blended in an amount of preferably 0.1-50 mass % based on the mass of the total composition in the adhesive layer.

As a copolymer having N-vinyl-2-pyrrolidone as one of its constituents, which is used in the adhesive layer of a preparation of the invention, its species is not particularly limited as long as it is a copolymer in which N-vinyl-2-pyrrolidone as one component is copolymerized with another monomer; A copolymer with an acrylic acid derivative is preferably used. Examples include copolymer of polymer (TSR) contained in 2-ethylhexyl acrylate·vinylpyrrolidone copolymer solution (manufactured by Sekisui Kagaku Co., Ltd.) and 1, 6-hexaneglycol dimethacrylate, which are described in Drug Additives Encyclopedia 2000 (edited by Japan Drug Additives Society) as adhesive agents.

An acrylic polymer or a rubber polymer can be used for the adhesive layer in the preparations of the invention.

As the acrylic polymer there is no particular restriction as long as it is copolymerized with at least one of (meth) acrylic acid derivatives represented by 2-ethylhexyl acrylate, methyl acrylate, butyl acrylate, hydroxyethyl acrylate, 2-ethylhexyl methacrylate and the like; Adhesive agents such as acrylic acid·octyl acrylate copolymer, acrylate·vinyl acetate copolymer, 2-ethylhexyl acrylate·2-ethylhexyl methacrylate·dodecyl methacrylate, methyl acrylate·2-ethylhexyl acrylate copolymer resin emulsion and acrylic polymer contained in acrylic resin alkanolamine liquid, which are, for example, described in Drug Additives Encyclopedia 2000 (editedby Japan Drug Additives Society) as adhesive agents, DURO-TAK acrylic adhesive series (manufactured by National Starch and Chemical Co., Ltd.), Eudragit series (Higuchi Syoukai) and the like, can be used. Also, like TSR described above, an adhesive agent already containing N-vinyl-2-pyrrolidone as one of the constituents can reasonably be used as a base.

As the rubber polymer, illustrative are styrene-isoprene-styrene block copolymer (hereinafter abbreviated as SIS), isoprene rubber, polyisobutylene (hereinafter abbreviated as PIB), styrene-butadiene-styrene block copolymer (hereinafter abbreviated as SBS), styrene-butadiene rubber, polysiloxane and the like. Among them SIS and PIB are preferable, and SIS is in particular preferable.

The hydrophobic polymer as above may be used in a mixture of two or more species, and considering formation of the adhesive layer and sufficient permeability, the blending amount of the polymer based on the mass of the total composition may be 10-90 mass %, preferably 30-90 mass %, more preferably 30-70 mass %.

In the invention it is desired to have an organic acid be contained in the adhesive layer in the case that the form of a drug is a drugly acceptable acid-addition salt, and as organic acids used, illustrative are aliphatic (mono-, di-, tri-)carboxylic acids (e.g., acetic acid, propionic acid, iso-butylic acid, caproic acid, caprylic acid, lactic acid, maleic acid, pyruvic acid, oxalic acid, succinic acid, tartaric acid and the like), aromatic carboxylic acids (e.g., phthalic acid, salicylic acid, bezoic acid, acetyl salicylic acid and the like), alkyl sulfonic acids (e.g., methane sulfonic acid, ethane sulfonicacid, propyl sulfonic acid, butane sulfonic acid, polyoxyethylene alkyl ether sulfonic acid and the like) , alkyl sulfonic acid derivatives (e.g., N-2-hydroxyethyl-piperidine-N'-2-ethane sulfonic acid (hereinafter abbreviated as HEPES) and the like) and cholic acid (e.g., dehydrocholic acid and the like). Among them acetic acid, propionic acid, lactic acid and salicylic acid are preferable, and acetic acid is in particular preferable. In addition, these organic acids may be used as salts thereof or in a mixture with salts thereof.

Considering sufficient permeation amount as the adhesive preparation and the irritating properties to the skin, these organic acid can be blended preferably in the amount of 0.01-20 mass % based on the mass of the total composition of the adhesive layer, more preferably 0.1-15 mass %, in particular preferably 0.1-10 mass %.

An absorption promoter may be contained in the adhesive layer of the preparations of the invention, and as an absorption promoter, any compound in which an absorption promoting effect is shown may be used. Examples includes C₆-C₂₀ fatty acids, fatty alcohols, fatty acid esters, amides or ethers, aromatic organic acids, aromatic alcohols, aromatic fatty acid esters or ethers (these may be saturated or unsaturated, and may be cyclic, straight or branched), furthermore lactic acid esters, acetic acid esters, monoterpene compounds, sesquiterpene compounds, Azone, Azone derivatives, pyrothiodecane, glycerol fatty acid esters, propylene glycol fatty acid esters, sorbitan fatty acid esters (Span type), polysorbates (Tween type), polyethylene glycol fatty acid esters, polyoxyethylene hardened castor oils (HCO type), polyoxyethylene alkyl ethers, sucrose fatty acid esters, plant oils and the like.

Specifically, caprylic acid, capric acid, caproic acid, lauric acid, myristic acid, palmitic acid, stearic acid, isostearic acid, oleic acid, linoleic acid, linolenic acid, lauryl alcohol, myristyl alcohol, oleyl alcohol, isostearyl alcohol, cetyl alcohol, methy laurate, hexyl laurate, lauric acid diethanolamide, isopropyl myristate, myristyl myristate, dodecyl myristate, cetyl palmitate, salicylic acid, methyl salicylate, ethylene glycol salicylate, cinnamic acid, methyl cinnamate, cresol, cetyl lactate, lauryl lactate, ethyl acetate, propyl acetate, geraniol, thymol, eugenol, terpineol, 1-menthol, borneol, d-limonene, isoeugenol, isoborneol, nerol, dl-camphor, glycerol monocaprylate, glycerol monocaprate, glycerol monolaurate, glycerol monooleate, sorbitan monolaurate, sucrose monolaurate, polysorbate 20, propylene glycol, propylene glycol monolaurate, polyethylene glycol monolaurate, polyethylene glycol monostearate, polyoxyethylene lauryl ether, HCO-60, pyrothiodecane and olive oil are preferred, and lauryl alcohol, isostearyl alcohol, lauric acid diethanolamide, glycerol monocaprylate, glycerol monocaprate, glycerol monooleate, sorbitan monolaurate, propylene glycol monolaurate, polyoxyethylene lauryl ether and pyrothiodecane are particularly preferred.

The absorption promoter may be used in a mixture of two or more species, and considering sufficient permeability as the adhesive preparation and irritating properties such as rubor, edema and the like, it can be blended preferably in 0. 01-20 mass % based on the mass of the total composition of the adhesive layer, more preferably 0.05-10 mass %, in particular preferably 0.1-5 mass %.

A plasticizer may be contained in the adhesive layer of the preparations of the invention, and as usable plasticizers, illustrative are petroleum oils (e.g., paraffin type process oil, naphthalene type process oil, aromatic type process oil and the like), squalane, squalene, vegetable oils (e.g., olive oil, camellia oil, castor oil, tall oil, peanut oil), silicone oil, dibasic acid esters (e.g., dibutyl phthalate, dioctyl phthalate and the like), liquefied rubber (e.g., polybutene, liquefied isoprene rubber), liquefied fatty acid esters (isopropyl myristate, hexyl laurate, diethyl sebacate, diisopropylsebacate), diethylene glycol, polyethylene glycol, glycol salicylate, propylene glycol, dipropylene glycol, triacetin, triethyl citrate, crotamiton and the like. In particular, liquid paraffin, liquefied polybutene, crotamiton, diethyl sebacate and hexyl laurate are preferred.

These constituents may be used in a mixture of two or more species, and considering sufficient permeability and maintenance of sufficient agglutinative strength as the adhesive preparations, the blending amount of such plasticizers based on the total composition in the adhesive layer can be 10-70 mass % in total, preferably 10-60 mass %, more preferably 10-50 mass %.

A tackifying resin may be contained in the adhesive layer of the percutaneous absorption preparations of the invention in case of insufficient adhesive strength, and as usable tackifying resins, illustrative are rosin derivatives (e.g., rosin, glycerol esters of rosin, hydorogenated rosin, glycerol esters of hydorogenated rosin, pentaerythritol esters of rosin and the like), alicyclic saturated hydrocarbon resins (e.g., Arcon P 100, manufactured by Arakawa Kagaku Kogyo Co., Ltd.), aliphatic hydrocarbon resins (e.g., Quintone B170, manufactured by Nihon Zeon Co., Ltd.), terpene resins (e.g., Clearon P-125, manufactured by Yasuhara Drugs Co., Ltd.), maleic acid resins and the like. In particular, glycerol esters of hydorogenated rosin, alicyclic saturated hydrocarbon resins, aliphatic hydrocarbon resins and terpene resins are preferred.

Considering an enough adhesive strength as the adhesive preparations and the irritating properties to the skin at the time of dissection, the blend amount of such tackifying resins based on the total composition in the adhesive layer can be 5-70 mass %, preferably 5-60 mass %, more preferably 10-50 mass %.

Also, if required, antioxidants, fillers, cross-linking agents, preservatives or UV absorbers can be used. As antioxidants, tocopherol and its ester derivatives, ascorbic acid, ascorbic acid-stearic acid ester, nordihydroguaretic acid, dibutyl hydroxy toluene (BHT), butyl hyroxy anisole and the like are desirable. As fillers, calcium carbonate, magnesium carbonate, silicate (e.g., aluminum silicate, magnesium silicate and the like), silicic acid, barium sulfate, calcium sulfate, calcium zincate, zinc oxide, titanic oxide and the like are desirable. As cross-linking agents, thermosetting resins such as amino resins, phenol resins, epoxy resins, alkyd resins and unsaturated polyesters, isocyanate compounds, block isocyanate compounds, organic type cross-linking agents, and inorganic type cross-linking agents such as metals or metal compounds, are desirable. As preservatives, ethyl p-hydroxy benzoate, propyl p-hydroxy benzoate, butyl p-hydroxy benzoate and the like are desirable. As UV absorbers, p-amino benzoic acid derivatives, anthranilic acid derivatives, salicylic acid derivatives, coumarin derivatives, amino acid type compounds, imidazoline derivatives, pyrimidine derivatives, dioxane derivatives and the like are desirable.

Such antioxidants, fillers, cross-linking agents, preservatives and UV absorbers can be blended preferably in an amount of not more than 10 mass % in total based on the mass of the total composition in the adhesive layer of the adhesive preparations, more preferably not more than 5 mass % and in particular preferably not more than 2 mass %.

The drug-containing adhesive layer containing the composition described above can be prepared by any method. For example, a base composition containing a drug is heat-melted, coated on removable paper or a support body, followed by affixing to the support or the removable paper to give the present preparations. Also, base constituents containing a drug are dissolved in solvent such as toluene, hexane or ethyl acetate, spread on removable paper or a support body, dried to remove solvent, followed by affixing to the support or the removable paper to give the present preparations.

As to the support layer in Fig. 1, an elastic or a non-elastic support body can be used, and for example, it can be selected from fabric, nonwoven fabric polyurethane,polyester,polyvinyl acetate, polyvinylidene chloride, polyethylene, polyethylene terephthalate, aluminum sheet and the like, or composite materials thereof.

### [Example]

In the following, the invention is explained in more detail by the examples. The invention, however, is not limited to these examples, and various changes may be made without departing from the spirit of the invention. Further, in the examples, all % mean % by mass.

### (Example 1)

| | |
|---|---|
| SIS | 15.0% |
| TSR | 15.0% |
| Alicyclic saturated hydrocarbon resin (Arcon P 100) | 35.0% |
| Liquid paraffin | 16.0% |
| Sodium acetate | 3.0% |
| Acetic acid | 2.0% |
| Sorbitan monolaurate | 5.0% |
| Pergolide mesylate | 9.0% |
| Total amount | 100.0% |

Pergolide mesylate, sorbitan monolaurate, liquid paraffin, sodium acetate and acetic acid were beforehand put in a mortar, ground and mixed thoroughly, followed by being mixed with remaining constituents dissolved in toluene. After the mixture was coated on removable paper, solvent was removed by drying, followed by affixing a support body to give a matrix adhesive preparation of the invention.

### (Example 2)

| | |
|---|---|
| SIS | 14.0% |
| TSR | 6.0% |
| Hydrogenated rosin ester | 40.0% |
| Sodium acetate | 5.0% |
| Liquid paraffin | 25.0% |
| Lauric acid diethanolamide | 5.0% |
| Pergolide mesylate | 5.0% |
| Total amount | 100.0% |

Pergolide mesylate, sodium acetate, lauric acid diethanolamide and liquid paraffin were beforehand put in a mortar, ground and mixed thoroughly, followed by being mixed with remaining constituents dissolved in a mixed solvent of toluene and ethyl acetate. After the mixture was coated on removable paper, solvent was removed by drying, followed by affixing a support body to give a matrix adhesive preparation of the invention.

### (Example 3)

| | |
|---|---|
| SIS | 15.0% |
| TSR | 20.0% |
| Alicyclic saturated hydrocarbon resin | 39.0% |
| Diethyl sebacate | 14.0% |
| Lactic acid | 6.0% |
| Polyoxyethylene lauryl ether | 3.0% |
| Bromocriptine mesylate | 3.0% |
| Total amount | 100.0% |

Bromocriptine mesylate, lactic acid, polyoxyethylene lauryl ether and diethyl sebacate were beforehandput in a mortar, ground and mixed thoroughly, followed by being mixed with remaining constituents dissolved in toluene. After the mixture was coated on removable paper, solvent was removed by drying, followed by affixing a support body to give a matrix adhesive preparation of the invention.

### (Example 4)

| | |
|---|---|
| TSR | 64.0% |
| Diethyl sebacate | 20.0% |
| Acetic acid | 5.0% |
| Sodium acetate | 2.0% |
| Lauric acid diethanolamide | 3.0% |
| Pergolide mesylate | 6.0% |
| Total amount | 100.0% |

Pergolide mesylate, acetic acid, sodium acetate, lauric acid diethanolamide and diethyl sebacate were beforehand put in a mortar, ground and mixed thoroughly, followed by being mixed with ethyl acetate solution of TSR. After the mixture was coated on removable paper, solvent was removed by drying, followed by affixing a support body to give a matrix adhesive preparation of the invention.

### (Example 5)

| | |
|---|---|
| TSR | 68.0% |
| Isopropyl myristate | 20.0% |
| Lactic acid | 6.0% |
| Polyoxyethylene lauryl ether | 3.0% |
| Bromocriptine mesylate | 3.0% |
| Total amount | 100.0% |

Bromocriptine mesylate, lactic acid, sodium acetate, polyoxyethylene lauryl ether and isopropyl myristate were beforehand put in a mortar, ground and mixed thoroughly, followed by being mixed with ethyl acetate solution of TSR. After the mixture was coated on removable paper, solvent was removed by drying, followed by affixing a support body to give the matrix adhesive preparation of the invention.

### [Comparative Example]

### (Comparative examples 1-3)

Except using PIB instead of TSR, the other constituents and the experimental processes were identical to those in the Examples 1-3.

### (Comparative examples 4, 5)

Except using Duro-Tak387-2287, which is free from N-vinyl-2-pyrrolidone, instead of TSR, the other constituents and the experimental processes were identical to those in the examples 1-3.

### (Skin permeability test in hairless mice)

A back part skin of a hairless mouse was stripped, and the dermal side was placed to a receptor layer side and installed in a flow-through cell (5 cm²) in which warm water of 37°C was circulated around the outer part. Each of the adhesive preparations obtained in the examples 1-5 as well as the comparative examples 1-5 was coated on the stratum corneum side, and samplings were carried out at every one hour for 18 hours at a rate of 5 ml/hour (hr) using the physiological saline in the receptor layer. As to the receptor solutions obtained at every hour, the flow amounts were accurately measured, and the drug concentrations were measured by a high-performance liquid chromatography, followed by calculation of the permeation rate per hour to determine the skin permeation velocity per unit area at the steady state. The results are shown in Table 1. The apparatuses used are as follows.

### Flow through cell

Name: Penetration Cell
Manufacturer: Laboratory Glass Apparatus Inc. Sampling apparatus
Name: Retriever IV Fraction Collector
Manufacturer: ISCO Inc.

### Warm water circulator

Name: Riko THERMOSTAT
Manufacturer: Riko Kagaku Sangyo Co., Ltd.

### (Physical test of preparations)

As to the preparations obtained in the Examples 1-5 and the comparative examples 1-5, the adhesive strength was measured by a probe tack tester and a peel measuring instrument, and the agglutinative strength by using a creep measuring instrument. As a result, those having no problem in the physical properties were evaluated as ○ and those having a problem were evaluated as ×. The results are shown in Table 1.

**Table 1**

| | Skin permeation Velocity of drug (µg/cm²/hr) | Physical properties of preparations |
|---|---|---|
| Example 1 | 3.5 | ○ |
| Example 2 | 6.5 | ○ |
| Example 3 | 3.5 | ○ |
| Example 4 | 3.8 | ○ |
| Example 5 | 2.8 | ○ |
| Comparative example 1 | 2.5 | ○ |
| Comparative example 2 | 3.5 | × |
| Comparative example 3 | 1.2 | × |
| Comparative example 4 | 1.2 | ○ |
| Comparative example 5 | 0.5 | ○ |

As is evident from the results shown in Table 1, it was found that the preparations obtained in each Example of the invention were remarkably high in the skin permeation velocity of the drugs compared with the preparations obtained in each Comparative Example, and can sufficiently be tolerable to practical use also in terms of the physical properties of the preparations.

### [Industrial Applicability]

The composition of the invention has no skin irritating property and can dissolve even a hardly soluble drug. Therefore, using said composition a preparation containing the hardly soluble drug can be prepared without skin irritating properties and without problems of the stability of the drug and the preparation, and of physical properties of the preparation.

For example, according to the adhesive preparations of the invention, drugs can efficiently be absorbed into circulating blood via the skin. Also, a side effect of the gastrointestinal system observed in case of oral administration, and a side effect of the central nervous system which can occur due to a rapid increase of the blood concentration can be avoided. Further, they have a good sticking property to the skin, and are very effective as external preparations aiming at percutaneous application. Therefore, the invention makes it possible to manufacture more effective and less costly preparations in manufacturing industries of external preparations containing hardly soluble drugs and in the related industries.

## Claims

1. A composition enhancing percutaneous absorption of a drug, **characterized in that** said composition comprises a copolymer having N-vinyl-2-pyrrolidone as one of its constituents.

2. The composition according to claim 1, **characterized in that** said composition comprises a copolymer having N-vinyl-2-pyrrolidone as one of its constituents and that the drug is a drug having an ergoline skeleton.

3. The composition according to claims 1 or 2, **characterized in that** the copolymer is a copolymer of a (meth)acrylic acid derivative and N-vinyl-2-pyrrolidone.

4. The composition according to claim 3, **characterized in that** the (meth)acrylic acid derivative is 2-ethylhexyl acrylate.

5. The composition according to claim 3, **characterized in that** the (meth) acrylic acid derivative is 2-ethylhexyl acrylate and 1,6-hexaneglycol dimethacrylate.

6. The composition according to any one of claims 2-5, **characterized in that** the drug having an ergoline skeleton is pergolide and/or pharmaceutically acceptable acid-addition salts thereof.

7. The composition according to claim 6, **characterized in that** the acid-addition salt of pergolide is pergolide mesylate.

8. The composition according to any one of claims 2-6, **characterized in that** the drug having an ergoline skeleton is bromocriptine and/or pharmaceutically acceptable acid-addition salts thereof.

9. The composition according to claim 8, **characterized in that** the acid-addition salt of bromocriptine is bromocriptine mesylate.

10. The composition according to any one of claims 1-9, **characterized in that** it contains an organic acid and/or pharmaceutically acceptable acid-addition salts thereof.

11. The composition according to claim 10, **characterized in that** the organic acid is acetic acid, propionic acid, lactic acid or salicylic acid.

12. The composition according to claim 11, **characterized in that** the organic acid is acetic acid or lactic acid.

13. A percutaneous absorption preparation, **characterized in that** the preparation comprises a drug-containing adhesive layer comprising the composition according to any one of claims 1-12 and a support layer.
